(19) Europäisches Patentamt
European Patent Office
Office européen des brevets

(11) Veröffentlichungsnummer: **0 428 013 A2**

(12) # EUROPÄISCHE PATENTANMELDUNG

(21) Anmeldenummer: **90120875.1**

(22) Anmeldetag: **31.10.90**

(51) Int. Cl.[5]: **C07D 209/44**, C07D 487/22, C09B 47/00, C09B 67/50, //(C07D487/22,259:00,209:00, 209:00,209:00,209:00)

(30) Priorität: **13.11.89 DE 3937716**

(43) Veröffentlichungstag der Anmeldung: **22.05.91 Patentblatt 91/21**

(84) Benannte Vertragsstaaten: **BE CH DE FR GB IT LI**

(71) Anmelder: **BAYER AG**

**W-5090 Leverkusen 1 Bayerwerk(DE)**

(72) Erfinder: **Kaletta, Bernd, Dr.**
**Heymannstrasse 40**
**W-5090 Leverkusen 1(DE)**
Erfinder: **Rolf, Meinhard, Dr., Mobay Corporation**
**Dyes and Pigment-Department**
**Rushpark, Charlston(US)**
Erfinder: **Wolf, Walther, Dr.**
**Am Schochert 30**
**W-8379 Bischofsmais(DE)**
Erfinder: **Terrell, David Richard, Dr.**
**Diependaele 4**
**B-2548 Lint(BE)**

(54) **Heterocyclische Verbindungen und daraus erhältliche Porphyrin-Verbindungen.**

(57) Porphyrine der Formel

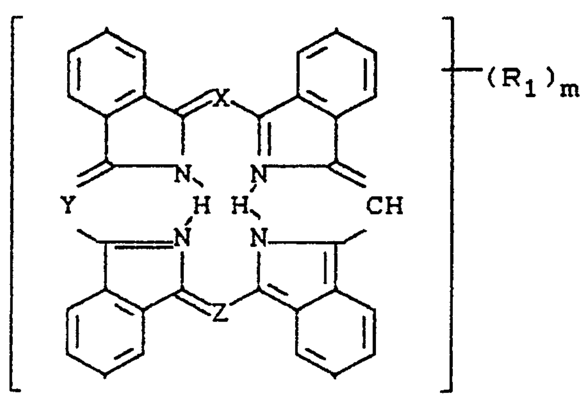

mit den in der Beschreibung angegebenen Substituentenbedeutungen erhält man durch Erhitzen von Verbindungen der Formel

EP 0 428 013 A2

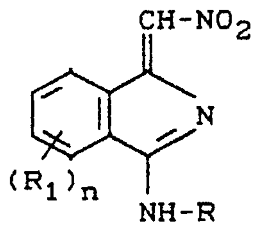

gegebenenfalls in Gegenwart von Verbindungen der Formel

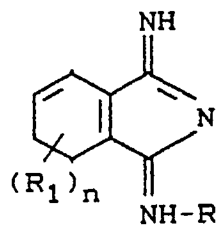

Die Porphyrine finden Verwendung als Pigmente.

## HETEROCYCLISCHE VERBINDUNGEN UND DARAUS ERHÄLTLICHE PORPHYRIN-VERBINDUNGEN

Gegenstand der vorliegenden Erfindung sind Verbindungen der Formel

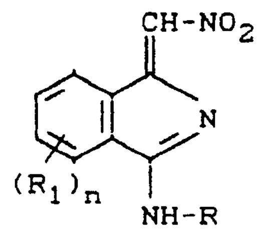

(I)

worin
R = H oder Substituent
$R_1$ = Substituent
n = 0 bis 2
sowie ein Verfahren zu deren Herstellung, dadurch gekennzeichnet, daß man Verbindungen der Formel

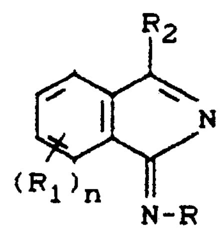

(II)

worin
$R_2$ = Halogen, Alkoxy oder NHR,
mit Nitromethan (III) umsetzt.

Die Substituenten $R_1$ können gleich oder verschieden sein.

Geeignete Substituenten R sind beispielsweise Alkyl, Aryl, Hetaryl, Acyl.

Geeignete Substituenten $R_1$ sind beispielsweise R, Alkoxy, Halogen, Carboxy, Carbonamid, Cyan, $NO_2$.

Zwei benachbarte Substituenten $R_1$ können auch zusammen mit den sie verbindenden C-Atomen des Benzolrings einen carbocyclischen oder heterocyclischen Ring bzw. Ringsystem bilden.

Alkyl steht vorzugsweise für $C_1$-$C_6$-Alkyl wie $CH_3$, $C_2H_5$, n- und i-$C_3H_7$ sowie n-, i- und t-$C_4H_9$.

Aryl steht vorzugsweise für solche carbocyclisch-aromatischen Reste, die 1, 2, 3 oder 4, insbesondere 1 oder 2 Ringe enthalten wie Phenyl, Diphenylyl und Naphthyl.

Hetaryl steht vorzugsweise für solche heterocyclischaromatischen Reste, die 1, 2, 3 oder 4, insbesondere 1 oder 2 fünf-, sechs- oder siebengliedrige Ringe enthalten, von denen mindestens einer 1, 2 oder 3, bevorzugt 1 oder 2 Heteroatome aus der Reihe O, N, S enthält. Als heterocyclisch-aromatische Reste seien beispielhaft genannt:
Pyridyl, Pyrimidyl, Pyrazinyl, Triazinyl, Furanyl, Pyrrolyl, Thienyl, chinolyl, Curarinyl, Benzofuranyl, benzoimidazolyl, Benzoxazolyl, Dibenzofuranyl, Benzothienyl, Dibenzothienyl, Indolyl, Carbazolyl, Pyrazolyl, Imidazolyl, Oxazolyl, Isooxazolyl, Thiazolyl, Indazolyl, Benzthiazolyl, Pyridazinyl, Cinnolyl, Chinazolyl, Chinoxalyl, Phthalazinyl, Phthalazindionyl, Phthalimidyl, Chromonyl, Naphtholactamyl, Benzopyridonyl, ortho-Sulfobenzimidyl, Maleinimidyl, Naphtharidinyl, Benzimidazolonyl, Benzoxazolonyl, Benzthiazoionyl, Benzthiazolinyl, Chinazolonyl, Pyrimidonyl, Chinoxalonyl, Phthalazonyl, Dioxapyrimidinyl, Pyridonyl, Isochinolonyl, Isochinolinyl, Isothiazolyl, Benzisoxazolyl, Benzisothiazolyl, Indazolonyl, Acridinyl, Acridonyl, Chinazolindionyl, Thinoxalindionyl, Benzoxazindionyl, Benzoxazinonyl und Naphthalimidyl.

Die Aryl- und Hetarylreste können übliche Substituenten aufweisen, z.B. F, Cl, Br, Alkyl, Alkoxy, gegebenenfalls substituiertes Amino, Carboxy, Carbonester, Carbonamid, Sulfo, Sulfonamid, Alkoxycarbonyl, Sulfonylamino, Alkylsulfonyl, Acylamino, Aminocarbonyloxy.
Halogen steht für F, Cl, Br, I.

Alkoxy ist bevorzugt $C_1$-$C_6$-Alkoxy.

Acyl steht bevorzugt für Alkyl- (bevorzugt $C_1$-$C_4$-Alkyl) oder gegebenenfalls substituiertes Phenylcarbonyl.

Bevorzugte Verbindungen (I) sind solche mit n = 0 sowie solche mit n = 1 und $R_1$ = Phenyl oder $OCH_3$ wobei jeweils R = H, Acetyl, Benzoyl darstellen, weiterhin solche mit n = 2, wobei zwei Reste $R_1$ zusammen mit den sie verbindenden C-Atomen einen Benzolring darstellen.

Die Umsetzung von (II) mit (III) wird vorzugsweise in einem inerten Verdünnungsmittel bei Temperaturen von 20 bis 150° C, gegebenenfalls in Gegenwart von Inertgasen durchgeführt.

Geeignete Verdünnungsmittel sind insbesondere wassermischbare organische Lösungsmittel wie Alkohole oder deren Ether beispielsweise Ethanol, Methanol, Butanol, Ethylenglykolmono-$C_1$-$C_4$-alkylether, Carbonsäuren wie Essigsäure; Dioxan, Pyridin, cyclische Amide wie Dimethylformamid.

Verbindungen (I) (die angegebene Formel ist die einer der möglichen tautomeren Formen) sind wertvolle Zwischenprodukte für die Herstellung von Pigmenten.

Gegenstand der Erfindung ist auch ein Verfahren zur Herstellung von Porphyrinen bzw. Gemischen von Porphyrinen der Formel

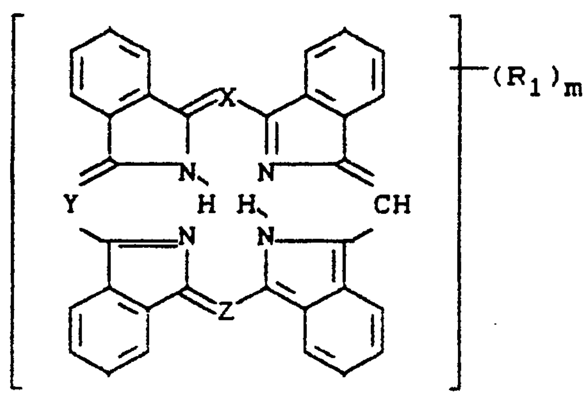

(IV)

worin

x, Y, Z = unabhängig voneinander CH oder N bedeuten, jedoch mindestens ein X, Y oder Z für N steht,

$R_1$ die oben angegebene Bedeutung besitzt und

m = 0 bis 8,

dadurch gekennzeichnet, daß (I), gegebenenfalls in Mischung mit bis zu 20 Mol (V)

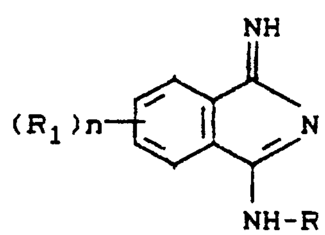

(V)

auf Temperaturen von 100 bis 300° C, vorzugsweise 150 bis 270° C, erhitzt.

Das Verfahren liefert einheitliche Verbindungen (IV) oder Gemische von unterschiedlichen Verbindungen (IV).

Man führt die Umsetzung vorzugsweise in einem Lösungsmittel durch. Als Lösungsmittel eignen sich beispielsweise Alkohole wie Pentanol, 2-Methyl-2-butanol, 2-Methyl-2-pentanol, 3-Methyl-3-butanol, ferner dipolaraprotische Lösungsmittel wie Dimethylformamid, Dimethylacetamid, Dimethylsulfoxid, N-Methylpyrrolidon, sowie aliphatische oder aromatische Kohlenwasserstoffe die gegebenenfalls durch Alkyl, Alkoxy, Halogen oder Nitrogruppen substituiert sein können wie Decalin, Xylol, Metsitylen, Chlorbenzol, Dichlorbenzol, Trichlorbenzol, Naphthalin, 1-Methylnaphthalin, 2-Methylnaphthalin, Chlornaphthalin, 2-Chlornaphthalin und gegebenenfalls substituierte heterocyclische Lösungsmittel wie Pyridin, Picolin, Chinolin.

Die Pigmente des Typs (IV) fallen nach dem oben beschriebenen Verfahren in ausreichender Reinheit an und können direkt oder nach geeigneter Formierung als Pigmente eingesetzt werden. Aufgrund ihrer guten Pigmenteigenschaften eignen sich Pigmente der Formel (IV) für die verschiedensten Pigmentapplikationen sowie als organische Fotoleiter beispielsweise für die Elektrofotografie.

So können sie zur Herstellung von sehr echt pigmentierten Systemen, wie Mischungen mit anderen Stoffen, Zubereitungen, Anstrichmitteln, Druckfarben, gefärbtem Papier und gefärbten makromolekularen Stoffen verwendet werden. Unter Mischung mit anderen Stoffen können z.B. solche mit anorganischen Weißpigmenten wie Titandioxid (Rutil) verstanden werden. Zubereitungen sind z.B. Flushpasten mit organischen Flüssigkeiten und gegebenenfalls Konservierungsmitteln. Die Bezeichnung Anstrichmittel steht z.B. für physikalisch oder oxidativ trocknende Lacke, Einbrennlacke, Reaktionslacke, Zweikomponentenlacke, Dispersionsfarben für wetterfeste Überzüge und Leimfarben. Unter Druckfarben sind solche für den Papier-, Textil- und Blechdruck zu verstehen.

Insbesondere eignen sich die neuen Pigmente zum Pigmentieren von makromolekularen organischen Stoffen. Die makromolekularen Stoffe können natürlichen Ursprungs sein wie Kautschuk, durch chemische Modifikation erhalten werden wie Acetylcellulose, Cellulosebutyrat oder Viskose oder synthetisch erzeugt werden wie Polymerisate, Polyadditionsprodukte und Polykondensate. Genannt seien plastische Massen wie Polyvinylchlorid, Polyvinylacetat, Polyvinylpropionat, Polyolefine, z.B. Polyethylen oder Polyamide, Superpolyamide, Polymerisate und Mischpolymerisate aus Acrylestern oder Methacrylestern, Acrylnitril, Acrylamid, Butadien, Styrol sowie Polyurethane und Polycarbonate. Die mit den beanspruchten Produkten pigmentierten Stoffe können in beliebiger Form vorliegen. Wegen ihrer hohen Wetterechtheit eignen sich die Pigmente der Formel (I) besonders zum Einsatz in Automobillacken, insbesondere für Metalliclackierungen.

Die Pigmente der Formel (IV) sind ausgezeichnet wasserecht, ölecht, säureecht, kalkecht, alkaliecht, lösungsmittelecht, überlackierecht, überspritzecht, sublimierecht, hitzebeständig, vulkanisierbeständig, sehr ergiebig, in plastischen Massen gut verteilbar und insbesondere ausgezeichnet lichtecht und migrationsecht.

Verbindungen der Formel (IV) sind z.T. bekannt (z.B. (IV) mit m = O, X, Y, Z = N). So wird in der GB-PS 494 738 folgendes Verfahren beschrieben; Methylenphthalimidinessigsäure wird mit Phthalodinitril in Gegenwart von Magnesiumspänen im Chlornaphthalin zum Magnesiumtetrabenzotriazaporphin umgesetzt. Das Magnesiumtetrabenzotriazaporphin wird in konzentrierter Schwefelsäure gelöst und anschließend auf Wasser ausgetragen, hierbei wird das metallfreie Tetrabenzotriazaporphin erhalten. Das nach dieser Vorschrift hergestellte Tetrabenzotriazaporphin liegt in der $\alpha$-Modifikation vor, die in der Röntgenbeugungsaufnahme bei den Bragg-Winkeln 6,7, 13,6, 15,0, 24,5 und 26,9 starke Banden zeigt.

Dieses Verfahren zur Herstellung von Verbindungen der Formel (IV) ist sehr umständlich, da zunächst die Methylenphthalimidinessigsäure in einer zweistufigen Synthese hergestellt werden muß. Zudem ist das metallfreie Tetrabenzotriazaporphin nur über den Magnesiumkomplex durch Umfällen aus Schwefelsäure zugänglich, was eine teure Aufarbeitung oder Entsorgung der Schwefelsäurelösung zur Folge hat.

Ein weiteres Verfahren zur Hestellung des Tetrabenzotriazaporphins wird von Barrett, Linstaed and Tuey (J. Chem. Soc. 1939, S. 1809-1822) durch Umsetzung von Phthalodinitril mit Methylmagnesiumiodid oder Methyllithium in Ether beschrieben.

Das nach dieser Vorschrift hergestellte Tetrabenzotriazaporphin liegt in der $\beta$-Modifikation vor, die in der Röntgenbeugungsaufnahme bei den Bragg-Winkeln 7,0; 8,9; 17,9; 20,3; 23,7; 26,0; 26,8; 27,7 und 30,0 starke Banden zeigt.

Ein Nachteil dieses Verfahrens liegt in der Verwendung von Ether als Lösungsmittel. Aufgrund des niedrigen Zündpunktes und der Neigung, explosive Peroxide zu bilden, ist Ether nur mit hohen Sicherheitsauflagen als Lösungsmittel in der Technik einsetzbar. Zudem sind metallorganische Reagenzien wie Methyllithium und Methylmagnesiumiodid aufgrund ihrer sehr hohen Reaktivität und der Neigung, mit Feuchtigkeit explosive Gase zu entwickeln, teuer und kann nur in speziellen Anlagen umgesetzt werden.

Demgegenüber geht das erfindungsgemäße Verfahren von gut zugänglichen Ausgangsmaterialien aus und die Produkte der allgemeinen Formel (IV) können in einer einstufigen Synthese direkt in metallfreier Form hergestellt werden.

Erhitzen von (I) mit n = 0 und R = H oder Acyl mit (V) mit n = 0 und R = H oder Acyl im Molverhältnis von etwa 1:1 liefert das Tetrabenzotriazaporphin

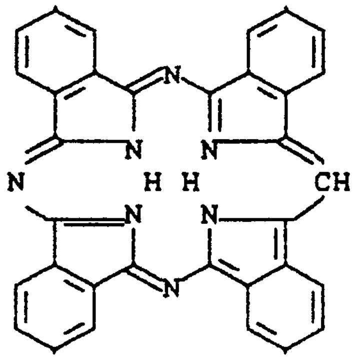

(IVa)

in der bekannten β-Modifikation.

Durch Mahlen der β-Modifikation der Verbindung der Formel (IVa), gegebenenfalls in Gegenwart eines anorganischen oder organischen Salzes, erhält man die neue ω-Modifikation der Verbindung der Formel (IVa), die in der Röntgenbeugungsaufnahme starke Banden bei den Bragg-Winkein 8,5; 10,2; 11,9; 14,4; 18,0; 20,4; 22,6; 24,0; 24,7 und 29,8 zeigt und die ebenfalls Gegenstand der Erfindung sind.

Die Mahlung erfolgt bevorzugt in Gegenwart von Salzen, insbesondere anorganischen Salzen, beispielsweise den Alkalisalzen starker anorganischer Säuren wie NaCl, vorzugsweise bei Raumtemperatur.

Ein weiteres Verfahren zur Herstellung der ω-Modifikation der Verbindung der Formel (IVa) besteht darin, die α-Modifikation der Verbindung der Formel (IVa) durch Erhitzen auf 100 bis 300° C, bevorzugt in Gegenwart eines Lösungsmittels, zu erhitzen.

Als Lösungsmittel eignen sich organische Lösungsmittel, beispielsweise Alkohole, Phenole, dipolar-aprotische Lösungsmittel wie Dimethylformamid, Dimethylacetamid, Dimethylsulfoxid, N-Methylpyrrolidon, sowie bevorzugt aliphatische oder aromatische Kohlenwasserstoffe, die gegebenenfalls durch Alkyl, Acyl, Alkoxy, Halogen, Ester oder Nitrogruppen substituiert sein können wie Decalin, Xylol, Metsitylen, Chlorbenzol, Dichlorbenzol, Trichlorbenzol, Naphthalin, 1-Methylnaphthalin, Methylbenzoat, 2-Methylnaphthalin, 1-Chlornaphthalin, 2-Chlornaphthalin und gegebenenfalls substituierte heterocyclische Lösungsmittel wie Pyridin, Picolin, Chinolin.

Ein weiteres Verfahren zur Herstellung der ω-Modifikation der Verbindung der Formel (IVa) besteht darin, die Pigmentsynthese nach dem erfindungsgemäßen Herstellungsverfahren in Gegenwart von vorzugsweise 0,001 bis 0,1 Mol der ω-Modifikation der Verbindung der Formel (IVa) bezogen auf 1 Mol der Verbindung der Formel (I) durchzuführen.

Die ω-Modifikation der Verbindung der Formel (IVa) ist aufgrund ihrer besonders guten Pigmenteigenschaften für die verschiedensten obengenannten Pigmentapplikationen sowie als organisches Fotoleiter-Pigment verwendbar.

## Beispiel 1

72,5 g Aminoiminoisoindolenin und 81 ml Nitromethan werden in 400 ml Methanol 12 Stunden zum Rückfluß erhitzt. Die gebildeten Kristalle werden kalt abgesaugt und mit Methanol gewaschen. Man erhält 62 g einer Verbindung, die in einer ihrer tautomeren Formen der Formel

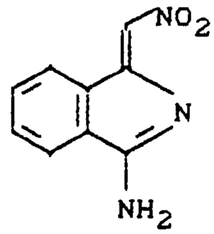

entspricht.

Beispiel 2

Man verfährt wie in Beispiel 1, leitet jedoch während der Reaktion einen schwachen Luftstrom durch die Lösung. Ausbeute: 68 g der in Beispiel 1 erhaltenen Verbindung.

Beispiel 3

Man verfährt wie in Beispiel 1, setzt jedoch anstelle des Aminoiminoisoindolenins 87,5 g des 5-Methoxy-aminoiminoisoindolenins ein. Es werden 63 g einer Verbindung erhalten, die in einer ihrer tautomeren Formen der Formel

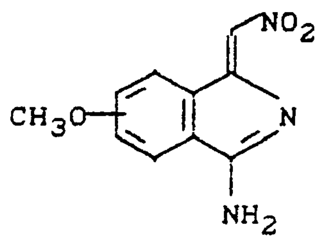

entspricht.

Beispiel 4

Man verfährt wie in Beispiel 1, setzt jedoch anstelle des Aminoiminoisoindolenins 97,5 g des Benzo-aminoiminoisoindolenins ein. Es werden 94,4 g einer Verbindung erhalten, die in einer ihrer tautomeren Formen der Formel

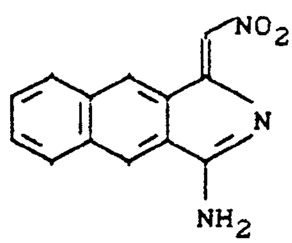

entspricht.

Beispiel 5

500 ml 1-Methylnaphthalin werden in einer Destillationsapparatur auf 240° C erhitzt. Binnen 30 Minuten wird eine Suspension aus 189 g der Verbindung aus Beispiel 1 und 145 g Aminoiminoisoindolenin in 700 ml 1-Methylnaphthalin zugetropft, das Reaktionswasser wird kontinuierlich abdestilliertl Anschließend wird 10 Stunden bei 240° C nachgerührt, die entstandenen Kristalle werden kalt abgesaugt und gründlich mit DMF und Methanol gewaschen. Es werden 102 g der Verbindung der Formel

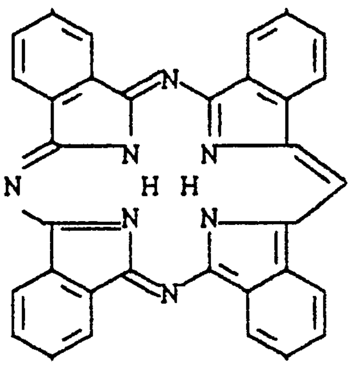

in der β-Modifikation erhalten.

Beispiel 6

71 g der Verbindung aus Beispiel 1, 55 g Aminoiminoisoindolenin und 500 ml Nitrobenzol werden 3 Stunden zum Rückfluß erhitzt. Durch kaltes Absaugen und gründliches Waschen mit DMF und Methanol werden 31,2 g der Verbindung aus Beispiel 5 in der β-Modifikation erhalten.

Beispiel 7

21,9 g der Verbindung aus Beispiel 3 und 14,5 g Aminoiminoisoindolenin werden in 100 ml o-Dichlorbenzol 12 Stunden zum Rückfluß erhitzt. Durch kaltes Absaugen und Waschen mit Methanol erhält man 13,2 g der Verbindung mit der Formel

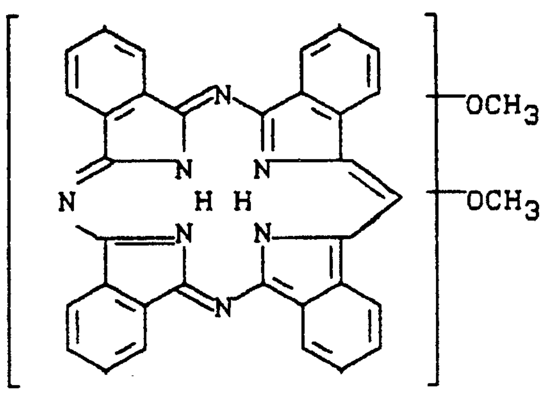

Beispiel 8

28,4 g der Verbindung aus Beispiel 1 und 26,3 g 5-Methoxy-aminoiminoisoindolenin werden in 100 ml Xylol 24 Stunden zum Rückfluß erhitzt. Die Kristalle werden abgesaugt, in DMF ausgerührt und mit Methanol gewaschen. Es werden 21,3 g der Verbindung aus Beispiel 7 erhalten.

Beispiel 9

56,7 g der Verbindung aus Beispiel 1 werden in 250 ml Amylalkohol 12 Stunden zum Rückfluß erhitzt. Die Kristalle werden kalt abgesaugt und mit DMF und Ethanol gewaschen. Es werden 5,3 g eines Gemisches aus der Verbindung aus Beispiel 5, Tetrabenzodiazaporphin und dem Tetrabenzomonoazaporphin erhalten.

Beispiel 10

38,7 g der Verbindung aus Beispiel 1 und 14,5 g Aminoiminoisoindolenin werden in 100 ml Chlornaphthalin 2 Stunden zum Rückfluß erhitzt. Die erhaltenen Kristalle werden abgesaugt und mit Aceton gewaschen. Es werden 9,3 g eines Gemisches aus der Verbindung aus Beispiel 5 und Tetrabenzodiazaporphin erhalten.

Beispiel 11

18,9 g der Verbindung aus Beispiel 1 und 43,5 g Aminoiminoisoindolenin werden in 100 ml Nitrobenzol 12 Stunden zum Rückfluß erhitzt. Nach dem kalten Absaugen werden 16,4 g einer Mischung aus Phthalocyanin und der Verbindung aus Beispiel 5 erhalten.

Beispiel 12 (Anwendungsbeispiel)

4 g feingemahlenes Pigment gemäß Beispiel 5 werden in 92 g eines Einbrennlackes folgender Zusammensetzung dispergiert:
33 % Alkydharz
15 % Melaminharz
5 % Glykolmonomethylether
34 % Xylol
13 % Butanol

Als Alkydharze kommen Produkte auf Basis synthetischer und pflanzlicher Fettsäuren wie Kokosöl, Rizinusöl, Rizinenöl, Leinöl u.a. in Frage. Anstelle von Melaminharzen können Harnstoffharze verwendet werden.

Nach erfolgter Dispergierung wird der pigmentierte Lack auf Papier-, Glas-, Kunststoff- oder Metall-Folien aufgetragen und 30 Minuten bei 130 °C eingebrannt, wobei man eine grüne Lackierung erhält.

Beispiel 13 (Anwendungsbeispiel)

0,2 g des nach Beispiel 5 erhaltenen Pigmentes werden bei 160 °C auf einem Mischwalzwerk in 65 g stabilisiertem PVC und 35 g Diisooctylphthalat dispergiert und bei 160 °C ausgewalzt. Man erhält eine grüne Folie von sehr guter Licht- und Migrationsechtheit.

Beispiel 14

10 g Tetrabenzotriazaporphin in der $\beta$-Modifikation, hergestellt z.B. nach Beispiel 5, 90 g Kochsalz und 600 g Stahlkugeln werden in einer Schwingmühle 10 Stunden gemahlen, anschließend mit heißem Wasser ausgerührt und salzfrei gewaschen. Es werden 9,9 g des Tetrabenzotriazaporphins in der neuen $\omega$-Modifikation erhalten.

Beispiel 15

9 g des Tetrabenzotriazaporphins aus Beispiel 14 werden in 100 ml Methylbenzoat 10 Stunden bei 150 °C gerührt. Das Produkt wird Kalt abgesaugt, mit Methanol gewaschen und getrocknet. Es werden 8,8 g Tetrabenzotriazaporphin in der $\omega$-Modifikation erhalten, die im Lack ein erhöhtes Deckvermögen zeigen.

Beispiel 16

10 g Tetrabenzotriazaporphin in beliebiger Modifikation werden in 100 g konz. Schwefelsäure gelöst, die Lösung wird binnen 30 Minuten in 900 ml Wasser ausgetragen. Das fein ausfallende Produkt wird abgesaugt mit Wasser sulfatfrei gewaschen und bei 50 °C in einem Umlufttrockenschrank getrocknet. Es

werden 9,5 g Tetrabenzotriazaporphin in der α-Modifikation erhalten.

Beispiel 17

9,5 g Tetrabenzotriazaporphin in der α-Modifikation, hergestellt nach Beispiel 16, werden in 100 ml Methyl benzoat suspendiert und 10 Stunden bei 180 °C gerührt. Das Produkt wird kalt abgesaugt, gut mit Methanol gewaschen und getrocknet. Es werden 9 g Tetrabenzotriazaporphin in der ω-Modifikation erhalten.

Beispiel 18

Man verfährt nach Beispiel 5, setzt jedoch 5 g der Verbindung aus Beispiel 14 als Impfkristalle zu. Es werden 110 g des Tetrabenzotriazaporphins in der ω-Modifikation erhalten.

Beispiel 19 (Anwendungsbeispiel)

4 g feingemahlenes Pigment gemäß Beispiel 14 werden in 92 g eines Einbrennlackes folgender Zusammensetzung dispergiert:
33 % Alkydharz
15 % Melaminharz
5 % Glykolmonomethylether
34 % Xylol
13 % Butanol

Als Alkydharze kommen Produkte auf Basis synthetischer und pflanzlicher Fettsäuren wie Kokosöl, Rizinusöl, Rizinenöl, Leinöl u.a. in Frage. Anstelle von Melaminharzen können Harnstoffharze verwendet werden.

Nach erfolgter Dispergierung wird der pigmentierte Lack auf Papier-, Glas-, Kunststoff- oder Metall-Folien aufgetragen und 30 Minuten bei 130 °C eingebrannt, wobei man eine grüne Lackierung erhält.

Beispiel 20 (Anwendungsbeispiel)

6 Teile Pigment nach Beispiel 14 werden in 12 Teilen Xylol, 4,1 Teilen Butylacetat und 0,7 Teilen n-Butanol mit 22,5 Teilen einer 20 %igen Lösung von Celluloseacetobutyrat in Butylacetat/Xylol (2:1) 30 Minuten im Red Devil mit 2 bis 3 mm Glaskugeln dispergiert. Nach Auffetten durch Zusatz von 10 Teilen eines gesättigten Polyesterharzes (Dynapol H 700) 7,3 Teilen Melaminharz 8,7 Teilen einer 20 %igen Lösung von Celluloseacetobutyrat in Butylacetat/Xylol (2:1), 18 Teilen Butylacetat, 1,6 Teilen n-Butanol und 9,7 Teilen Xylol wird nochmals 5 Minuten dispergiert.

Zu diesem Lack setzt man eine Dispersion von Aluminiumpaste (60 %) in einem organischen Lösungsmittel (ca. 1:2) in der Menge zu, daß das Verhältnis Pigment:Al zwischen 80:20 und 1:99 zu liegen kommt.

Dieser Lack wird aufgestrichen und nach dem Trocknen mit einem Klarlack auf Basis Acrylat/Melaminharz, der weitere Hilfsmittel wie z.B. UV-Absorber enthalten kann, überzogen und eingebrannt.

Man erhält eine grüne Metalliclackierung mit ausgezeichneter Wetterechtheit.

Beispiel 21

0,2 g des nach Beispiel 14 erhaltenen Pigmentes werden bei 160 °C auf einem Mischwalzwerk in 65 g stabilisiertem PVC und 35 g Diisooctylphthalat dispergiert und bei 160 °C ausgewalzt. Man erhält eine grüne Folie von sehr guter Licht- und Migrationsechtheit.

**Ansprüche**

1. Verbindungen der Formel

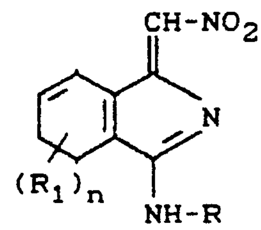

(I)

worin
R = H oder Substituent
$R_1$ = Substituent
n = 0 bis 2.

2. Verfahren zur Herstellung der Verbindungen des Anspruchs 1, dadurch gekennzeichnet, daß man eine Verbindung der Formel

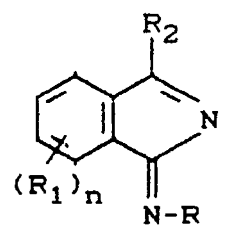

(II)

worin
R, $R_1$ und n die in Anspruch 1 angegebene Bedeutung haben und
$R_2$ Halogen, Alkoxy oder NHR ist, mit Nitromethan umsetzt.

3. Verfahren zur Herstellung von Porphyrinen bzw. Gemischen von Porphyrinen der Formel

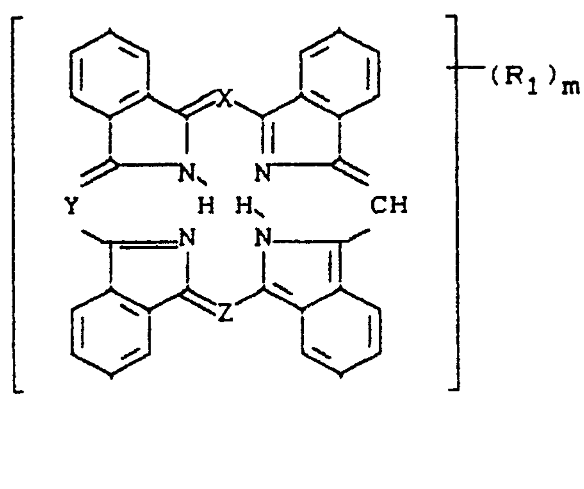

(IV)

worin
X, Y, Z = unabhängig voneinander CH oder N bedeuten, jedoch mindestens ein X, Y oder Z für N steht,
$R_1$ die in Anspruch 1 angegebene Bedeutung besitzt und
m = 0 bis 8,
dadurch gekennzeichnet, daß man eine Verbindung der Formel

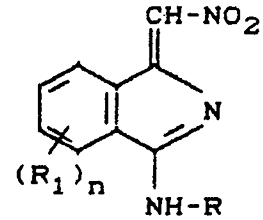

(I)

mit den in Anspruch 1 angegebenen Substituentenbedeutungen gegebenenfalls in Mischung mit bis zu 20 Mol einer Verbindung

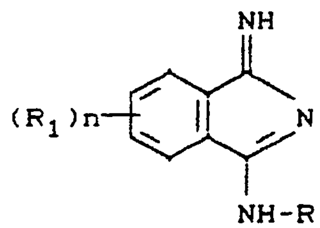

(V)

auf Temperaturen von etwa 100-300° C, vorzugsweise 150-270° C erhitzt.

4. ω-Modifikation der Verbindung der Formel

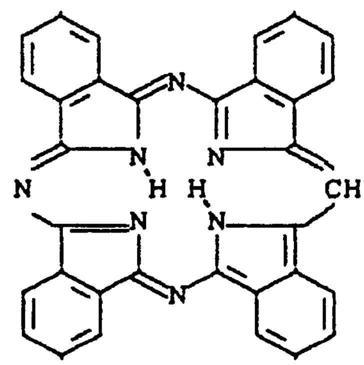

gekennzeichnet durch starke Banden in der Röntgenbeugungsaufnahme bei Bragg-Winkeln von 8,5; 10,2; 11,9; 14,4; 18,0; 20,4; 22,6; 24,0; 24,7 und 29,8.

5. Verwendung des Porphyrins des Anspruchs 4 als Pigment.

6. Mit dem Porphyrin des Anspruchs 4 pigmentierte organische Stoffe.

7. Verfahren zur Herstellung des Porphyrins des Anspruchs 4, dadurch gekennzeichnet, daß man die β-Modifikation des Porphyrins, bevorzugt in Gegenwart von Salzen wie den Alkalisalzen starker anorganischer Säuren, mahlt, oder die α-Modifikation - vorzugsweise in Gegenwart von Lösungsmitteln - auf etwa 100-300° C erhitzt.

8. Verfahren zur Herstellung des Porphins des Anspruchs 4, dadurch gekennzeichnet, daß man die Synthese in Gegenwart von Impfkristallen der ω-Modifikation durchführt.

9. Verwendung der Porphyrine gemäß Anspruch 3 als Pigment.

10. Mit den Porphyrinen gemäß Anspruch 3 pigmentierte organische Stoffe.